# EUROPÄISCHE PATENTSCHRIFT

(11) **EP 0 743 295 B1**
(45) Veröffentlichungstag und Bekanntmachung des Hinweises auf die Patenterteilung: **25.08.1999**
(21) Anmeldenummer: 96107061.2
(22) Anmeldetag: 06.05.1996
(51) Int. Cl.: C07C 35/12, B01J 23/76, B01J 23/78

(54) **Verfahren zur Herstellung von d,I-Menthol aus d-Menthol**
Process for preparing d,I-menthol from d-menthol
Procédé pour la préparation de d,I-menthol à partir de d-menthol

(30) Priorität: 17.05.1995 DE 19518023
(43) Veröffentlichungstag der Anmeldung: 20.11.1996
(73) Patentinhaber: BAYER AG, 51368 Leverkusen (DE)
(72) Erfinder: Darsow, Gerhard, Dr., 47804 Krefeld (DE); Petruck, Gerd-Michael, Dr., 40699 Erkrath (DE)

(56) Entgegenhaltungen:
- EP-A- 0 563 611
- DE-A- 2 314 813
- CATALYSIS LETTERS, Bd. 29, 1994, Seiten 57-67, XP002012695 ALLAKHVERDIEV ET AL.: "Liquid-phase stereoselective thymol hydrogenation over supported nickel catalysts"

## Beschreibung

Die Erfindung betrifft ein Verfahren zur Herstellung von d,l-Menthol durch katalytische Umlagerung von optisch aktivem d-Menthol in Gegenwart von Wasserstoff.

Unter den natürlich vorkommenden cyclischen Terpenalkoholen nimmt l-Menthol aufgrund seiner kühlenden und erfrischenden Wirkung eine Sonderstellung ein. l-Menthol ist der Hauptbestandteil des Pfefferminzöls und wird in der Riechstoff-, Geschmackstoff- und Arzneimittelindustrie eingesetzt.

Die Mentholherstellung durch katalytische Hydrierung von Thymol führt zum d,l-Racemat, das sich in optische Antipoden spalten läßt. Die 8 optisch aktiven Menthole unterscheiden sich in Bezug auf ihre organoleptischen Eigenschaften. l-Menthol hat einen charakteristischen Pfefferminzgeruch und die schon erwähnte erfrischende Wirkung. Das Racemat besitzt die vorteilhaften Eigenschaften des l-Menthols naturgemäß nur teilweise. Demzufolge bestand das Problem, das bei der Racematspaltung entstehende d-Menthol zu racemisieren, um aus dem neu entstandenen Racemat wieder l-Menthol gewinnen zu können.

Aus der US-PS 2 843 636 und der DE-AS 2 314 813 ist bekannt, daß man durch Erhitzen mit Wasserstoff in Gegenwart eines Kupferchromit- bzw. Cobalt/Mangan-Hydrierkatalysators u.a. d-Menthol racemisieren oder die Stereoisomeren des Menthols zu d,l-Menthol isomerisieren kann. Laut EP-A 563 611 kann man die Stereoisomeren des Menthols in Gegenwart von Wasserstoff an einem Festbettkatalysator, der auf einem mit einem Metall der Seltenen Erden und mit Mangan dotierten Träger Palladium, Ruthenium, Rhodium oder ein Gemisch dieser Elemente als Aktivbestandteile und Alkalimetallhydroxide und/oder -sulfate als Promotoren enthält, zu d,l-Menthol umlagern.

Diese Verfahren des Standes der Technik erzeugen entweder zu viele Nebenprodukte (die insbesondere bei einer kontinuierlichen Verfahrensweise durch Anreicherung störend ins Gewicht fallen), oder die verwendeten Katalysatoren verlieren zu rasch ihre Anfangsaktivität, sind von eingeschränkter mechanischer Stabilität, sind nur begrenzt belastbar und/oder erschweren eine Wiederaufarbeitung der gebrauchten Katalysatoren.

Es bestand daher der Wunsch, hoch belastbare und langlebige Katalysatoren für die Racemisierung von d-Menthol zu d,l-Menthol bereitzustellen, die frei von komplizierten Trägersystemen und daher wieder aufarbeitbar sein sollten.

Überraschenderweise läßt sich das geschilderte Problem mit Hilfe trägerfreier Festbettkatalysatoren lösen, die durch Reduktion von Formkörpern aus verpreßten Metall(hydr)oxidpulvern erhalten werden können. "Metall(hydr)oxide" im Sinne dieser Erfindung sind Metallhydroxide und/oder -oxide.

Gegenstand der Erfindung ist also ein kontinuierliches Verfahren zur Herstellung von d,l-Menthol durch katalytische Umlagerung von optisch aktivem d-Menthol in Gegenwart von Wasserstoff unter erhöhtem Druck und bei erhöhter Temperatur, dadurch gekennzeichnet, daß man die Umlagerung bei einem Wasserstoffdruck von 50 bis 350 bar, vorzugsweise 100 bis 300 bar und Temperaturen von 200 bis 350°C, vorzugsweise 220 bis 290°C im Festbettverfahren über als Katalysatoren dienenden trägerfreien Formkörpern durchführt, die durch Reduktion von Formkörpern aus verpreßten Pulvern von Nickel-, Mangan- und Erdalkalimetall(hydr)oxiden und gegebenenfalls (Hydr)Oxiden von Elementen der V. und/oder VI. Nebengruppe des Periodensystems erhältlich sind.

Die Belastbarkeit der erfindungsgemäß zu verwendenden Katalysatoren ist beachtlich:

Kommen lediglich trägerfreie Formkörper aus verpreßten Pulvern von Ni-, Mn⁻ und Erdalkalimetall(hydr)oxiden zum Einsatz, so liegt die stündliche Katalysatorbelastbarkeit zwischen 400 und 1000 g d-Menthol pro Liter Katalysator. Kommen zusätzlich verpreßte Pulver von (Hydr)Oxiden von Elementen der V. und/oder VI. Nebengruppe des Periodensystems zum Einsatz, so kann die stündliche Katalysatorbelastung bis auf 1500 g d-Menthol pro Liter Katalysator erhöht und die Reaktionstemperatur um bis zu 50°C gesenkt werden.

Die Ni-Anteile (jeweils gerechnet als Metall) betragen 40 bis 60 Gew.-%, die Mn-Anteile 10 bis 20 Gew.-%, die Erdalkalimetall-Anteile 0,2 bis 5 Gew.-% und die Anteile an Elementen der V. und/oder VI. Nebengruppe des Periodensystems insgesamt bis zu 5 Gew.-%, vorzugsweise 0,5 bis 5 Gew.-%. Der Rest zu 100 Gew.-% ist Sauerstoff für die in oxidischer Form vorliegenden Verbindungen.

Von den Erdalkalielementen eignen sich vor allem Magnesium, Calcium, Strontium und Barium, vorzugsweise Strontium und Barium. Von den Elementen der V. Nebengruppe eignen sich vorzugsweise Vanadium, Niob und Tantal, von den Elementen der VI. Nebengruppe vorzugsweise Chrom, Molybdän und Wolfram. Die als Promotoren wirkenden Elemente der V. und VI. Nebengruppe können entweder einzeln oder als Mischung mehrerer dieser Elemente zum Einsatz kommen.

Die Herstellung der trägerfreien Formkörper kann nach gebräuchlichen Methoden durch Verpressen der Metall(hydr)oxid-Pulvermischungen (gegebenenfalls nach vorausgegangener Temperierung bei höheren Temperaturen), beispielsweise auf Tablettier- oder Pelletier-Maschinen, unter hohem Druck erfolgen, wobei zur Verbesserung des Haftvermögens der Metall(hydr)oxidpartikel auch Graphit und/oder Klebstoffe in Mengen von 0,5 bis 3 Gew.-%, bezogen auf das Gesamtgewicht der zu verpressenden Bestandteile, zum Einsatz kommen können. Beispiele für Formkörper sind Tabletten, Kugeln oder Granulate mit Durchmessern von 3 bis 7 mm. Tablettierte Formkörper können weiterhin zur Vergrößerung der äußeren Oberfläche mit einer axialen Durchbohrung versehen sein. Solche Formkörper haben makroskopisch betrachtet eine glatte Oberfläche.

Die verpreßten Metall(hydro)oxid-Formkörper haben eine hohe Druckfestigkeit von 300 bis 800 N/cm², bevorzugt 400 bis 600 N/cm², auf die plane Formkörperoberfläche bzw. von 50 bis 200 N, bevorzugt 80 bis 140 N, auf die gewölbte Formkörperoberfläche. Die innere Oberfläche der verpreßten Metall(hydr)oxidpulver beträgt 30 bis 200 m²/g, bevorzugt 80 bis 160 m²/g. Die Druckfestigkeit der trägerfreien Formkörper kann nach DIN 50 106, die innere Oberfläche nach F.M. Nelsen und F.T. Eggertsen, Analyt. Chem. 30 (1958), 1387-1392 bzw. S.J. Gregg und S.W. Sing, Adsorption, Surface Area and Porosity, London 1982, Kap. 2 und 6 bestimmt werden.

Vor ihrem Einsatz müssen die Formkörper aus verpreßten (Hydr)Oxidpulvern sorgfältig reduziert werden. Dies geschieht vorzugsweise durch den Einsatz eines Reduktionsgases, das aus einer Inertgas/Wasserstoff-Mischung besteht, in der der Wasserstoffgehalt zu Beginn bei 10 bis 15 Vol.-% liegt. Als Inertgas wird bevorzugt Stickstoff eingesetzt. Die Reduktion erfolgt beispielsweise innerhalb eines Zeitraums von etwa 24 Stunden bei einer Reduktionstemperatur von 180 bis 220°C, wobei in der Endphase der Reduktion der Stickstoffanteil der Gasmischung mehr und mehr vermindert wird, bis diese schließlich aus einem Wasserstoff besteht. Die Reduktion ist beendet, wenn kein Wasserstoff mehr verbraucht und infolgedessen kein Reaktionswasser mehr gebildet wird.

Die Racemisierungsreaktoren können einzelne Hochdruckrohre aus Stahl oder einer Stahllegierung sein, die mit den Formkörpern ganz oder teilweise gefüllt werden, wobei bei größeren Rohrquerschnitten auch die Anwendung der trägerfreien Formkörper auf Horden (etwa Drahtkörbe oder ähnliches) nützlich sein kann; man kann jedoch auch Hochdruckrohrbündel innerhalb eines gemeinsamen Mantels anwenden, wobei die Einzelrohre wiederum mit den trägerfreien Formkörpern ganz oder teilweise gefüllt werden.

Das erfindungsgemäße Verfahren kann mit den im Festbett angeordneten Katalysatoren in der Gas- bzw. Rieselphase oder in der aufsteigenden Flüssigkeitsphase durchgeführt werden. Im allgemeinen wird bei molaren Wasserstoffüberschüssen gearbeitet, wobei mindestens die 5-fache molare Menge Wasserstoff pro Mol Ausgangsverbindung den Reaktor während des Verfahrensablaufs passiert.

Das erfindungsgemäße Verfahren kann mit oder ohne Lösungsmittel durchgeführt werden. Geeignete, unter den Reaktionsbedingungen inerte Lösungsmittel sind beispielsweise Methanol, Ethanol, Isopropanol.

Im Rahmen des erfindungsgemäßen Verfahrens sind sehr hohe Katalysatorstandzeiten von 20.000 bis 25.000 Stunden zu erreichen. Diese Standzeiten sind um ein vielfaches höher, als in früheren Veröffentlichungen (z.B. DE-AS 2 314 813) beschrieben.

Die beim erfindungsgemäßen Verfahren erfolgende Racemisierung läuft überraschenderweise so milde ab, daß die Bildung unverwertbarer Nebenprodukte, wie unerwünschter Kohlenwasserstoffe auf <0,5 Gew.-% gehalten wird.

Das erhaltene Reaktionsgemisch enthält einen so hohen Gehalt an d,l-Menthol, daß es durch einfache Destillation auf dieses gewünschte Produkt aufgearbeitet werden kann. In diesem Zusammenhang ist zu beachten, daß das Racemisierungsgleichgewicht bei 59,8 % l-Menthol liegt; die erfindungsgemäß erzielbaren Ausbeuten erreichen diesen Wert nahezu.

Nach der destillativen Abtrennung des gewünschten d,l-Menthols kann der Destillationsvorlauf mit dem Destillationssumpf wieder in die Reaktion zurückgeführt werden. Die dem destillativ entnommenen d,l-Menthol entsprechende Menge an Ausgangsmaterial wird ersetzt. Der im erfindungsgemäßen Verfahren nicht verbrauchte Wasserstoff kann im Kreis geführt werden.

Das erzeugte d,l-Menthol wird nach der Entfernung des Destillationsvorlaufes und des Destillationssumpfes in einer Reinheit von ≥ 99,9 Gew.-% erhalten und ist deshalb ohne weitere Reinigung für alle weiterverarbeitenden Prozesse einsetzbar.

Das nach der Destillation erhaltene farblose und glasklare Produkt hat einen Schmelzpunkt von 41°C und kann in Kristallisationsgeräten üblicher Bauart zur Kristallisation gebracht werden.

In den nachfolgenden Beispielen bedeutet die Bezeichnung "Nm³": Kubikmeter nach Umrechnung auf Normalbedingungen (1 bar, 25°C).

### Beispiele

### Beispiel 1

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstoff-frei gespült worden war, wurde mit 1,4 l durch Tablettierung von Pulvern von Nickel-, Mangan- und Barium-(hydr)oxiden hergestellter Formkörper gefüllt. Der Nickelgehalt der Tabletten lag bei 54 Gew.-%, der Mangangehalt bei 15 Gew.-%, der Bariumgehalt bei 1,5 Gew.-%. Die Tabletten hatten bei einer Zylinderhöhe von 5 mm und einem Durchmesser von 5 mm eine Druckfestigkeit von 450 N/cm² auf die plane Zylinderoberfläche und von 120 N auf die gewölbte Zylinderoberfläche sowie eine innere Oberfläche von 158 m²/g.

Die Tabletten wurden zunächst 6 Stunden im Stickstoffstrom getrocknet (Temperatur: max. 200°C, Menge: 5 Nm³ N₂/h). Die Aktivierung erfolgte unter einem Stickstoffdruck von 200 bar bei einer Temperatur zwischen 180 und 220°C, wobei dem Stickstoff allmählich Wasserstoff zugemischt wurde, dessen Zumischungsanteil anfangs 10 bis 15 Vol.-% betrug. Innerhalb von 24 Stunden wurde der Stickstoffanteil der Gasmischung mehr und mehr vermindert, bis schließlich reiner Wasserstoff den Reaktor durchströmte. Die Aktivierung war beendet, sobald sich kein Reaktionswasser im nachgeschalteten Abscheider mehr ansammelte.

Nach der Aktivierung des Katalysators wurde der Wasserstoffdruck im Reaktorsystem auf 300 bar erhöht. Anschließend wurden stündlich 1400 g d-Menthol (Reinheit: 99,9 Gew.-%) gemeinsam mit 10 Nm³ Wasserstoff unter einem Druck von 300 bar von oben nach unten durch das Hochdruckrohr gepumpt, wobei das d-Menthol vor Eintritt in das Hochdruckrohr in einem vorgeschalteten elektrisch beheizten Wärmeaustauscher auf eine Temperatur von 290°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Reaktionsystem zurückgeführt wurde, getrennt.

Der d-Menthol-Durchsatz entsprach einer Katalysatorbelastung von 1,0 kg/l Katalysator x h. Der Katalysator war auch nach einer Laufzeit von 7000 h noch hoch wirksam.

Nach der destillativen Entfernung der Vor- und Nachsieder wurde das erzeugte d,l-Menthol in einer Reinheit von 99,9 Gew.-% erhalten.

### Beispiel 2

Ein Hochdruckrohr wie in Beispiel 1 wurde unter Inertgas mit 1,4 l durch Tablettierung von Pulvern von Nickel-, Mangan-, Barium- und Wolfram(hydr)oxiden hergestellter Formkörper gefüllt. Der Nickelgehalt der Tabletten lag bei 48 Gew.-%, der Mangengehalt bei 15 Gew.-%, der Bariumgehalt bei 1,0 Gew.-% und der Wolframgehalt bei 0,6 Gew.-%. Die Tabletten hatten bei einer Zylinderhöhe von 5 mm und einem Durchmesser von 5 mm eine Druckfestigkeit von 545 N/cm² auf die plane Zylinderoberfläche und von 110 N auf die gewölbte Zylinderoberfläche sowie eine innere Oberfläche von 117 m²/g.

Nach der Aktivierung dieser verpreßten Metalloxid-Pulvermischung wie in Beispiel 1 wurde der Wasserstoffdruck auf 300 bar erhöht.

Anschließend wurden stündlich 1800 g d-Menthol (Reinheit: 99,9 Gew.-%) gemeinsam mit 10 Nm³ Wasserstoff unter einem Druck von 300 bar kontinuierlich von oben nach unten durch das Hochdruckrohr gepumpt, wobei das d-Menthol vor Eintritt in das Hochdruckrohr auf eine Temperatur von 240°C erhitzt wurde.

Der d-Menthol-Durchsatz entsprach einer Katalysatorbelastung von 1,29 kg/l Katalysator x h. Der Katalysator war auch nach einer Laufzeit von 6000 h noch hoch wirksam.

### Beispiel 3

Ein Hochdruckrohr wie in Beispiel 1 wurde unter Inertgas mit 1,4 l durch Tablettierung von Pulvern von Nickel-, Mangan-, Barium- und Molybdän(hydro)oxiden hergestellter Formkörper gefüllt. Der Nickelgehalt der Tabletten lag bei 60 Gew.-%, der Mangangehalt bei 15 Gew.-%, der Bariumgehalt bei 1,5 Gew.-% und der Molybdängehalt bei 1,0 Gew.-%. Die Tabletten hatten bei einer Zylinderhöhe von 5 mm und einem Durchmesser von 5 mm eine Druckfestigkeit von 635 N/cm² auf die plane Zylinderoberfläche und von 90 N auf die gewölbte Zylinderoberfläche sowie eine innere Oberfläche von 138 m²/g.

Nach der Aktivierung dieser verpreßten Metall(hydr)oxid-Pulvermischung wie in Beispiel 1 wurde der Wasserstoffdruck bei 200 bar belassen.

Anschließend wurden stündlich 1900 g d-Menthol gemeinsam mit 10 Nm³ Wasserstoff unter einem Druck von 200 bar kontinuierlich von oben nach unten durch das Hochdruckrohr gepumpt, wobei das d-Menthol und der Wasserstoff vor Eintritt in das Hochdruckrohr auf 230°C erhitzt wurden.

Der d-Menthol-Durchsatz entsprach einer Katalysatorbelastung von 1,36 kg/l Katalysator x h. Der Katalysator war auch nach einer Laufzeit von 7400 h noch hoch wirksam.

### Beispiel 4

Ein Hochdruckrohr wie in Beispiel 1 wurde unter Inertgas mit 1,4 l durch Tablettierung von Pulvern von Nickel-, Mangan-, Strontium- und Vanadium-(Hydr)oxiden hergestellter Formkörper gefüllt. Der Nickelgehalt der Tabletten lag bei 54 Gew.-%, der Mangangehalt bei 16 Gew.-%, der Strontiumgehalt bei 0,9 Gew.-% und der Vanadiumgehalt bei 1,2 Gew.-%. Die Tabletten hatten bei einer Zylinderhöhe von 5 mm und einem Durchmesser von 5 mm eine Druckfestigkeit von 691 N/cm² auf die plane Zylinderoberfläche und von 110 N auf die gewölbte Zylinderoberfläche sowie eine innere Oberfläche von 141 m²/g.

Nach der Aktivierung des Katalysators wie im Beispiel 1 wurde der Wasserstoffdruck auf 300 bar erhöht. Anschließend wurden stündlich 1800 g d-Menthol, gemeinsam mit 15 Nm³ Wasserstoff unter einem Druck von 300 bar kontinuierlich von oben nach unten durch das Hochdruckrohr gepumpt, wobei das d-Menthol vor Eintritt in das Hochdruckrohr auf eine Temperatur von 260°C erhitzt wurde.

Das das Reaktionsrohr verlassende Produkt wurde auf eine Temperatur <60°C abgekühlt und in einem Gasabscheider vom überschüssigen Wasserstoff, der wieder in das Reaktionssystem zurückgeführt wurde, getrennt.

Der Durchsatz des Reaktionsgemisches entsprach einer Katalysatorbelastung von 1,3 kg/l Katalysator x h. Der Katalysator war auch nach einer Laufzeit von 2000 h noch hoch wirksam.

## Patentansprüche

1. Kontinuierliches Verfahren zur Herstellung von d,l-Menthol durch katalytische Umlagerung von optisch aktivem d-Menthol in Gegenwart von Wasserstoff unter erhöhtem Druck und bei erhöhter Temperatur, dadurch gekennzeichnet, daß man die Umlagerung bei einem Wasserstoffdruck von 50 bis 350 bar und Temperaturen von 200 bis 350°C im Festbettverfahren über als Katalysatoren dienenden trägerfreien Formkörpern durchführt, die durch Reduktion von Formkörpern aus verpreßten Pulvern von Nickel-, Mangan- und Erdalkalimetall(hydr)oxiden und gegebenenfalls (Hydr)oxiden von Elementen der V. und/oder VI. Nebengruppe des Periodensystems erhältlich sind.

2. Verfahren nach Anspruch 1, wonach die für die Reduktion zu verwendenden Formkörper aus verpreßten Metall(hydro)oxidpulvern 30 bis 60 Gew.-% Nickel, 10 bis 20 Gew.-% Mangan, 0,2 bis 5 Gew.-% Erdalkalimetall und bis zu 5 Gew.-% Elemente der V. und/oder VI. Nebengruppe des Periodensystems (jeweils gerechnet als Metall) enthalten, wobei sich die Prozentangaben auf die Gesamtmenge Metall(hydr)oxid-Pulvergemisch beziehen und der Rest zu 100 Gew.-% Sauerstoff ist.

3. Verfahren nach Anspruch 1, wonach die Formkörper aus verpreßtem Metall(hydr)oxidpulver eine Druckfestigkeit von 300 bis 800 N/cm² auf die plane Formkörperoberfläche und von 50 bis 200 N auf die gewölbte Formoberfläche (gemessen nach DIN 50 106) besitzen.

4. Verfahren nach Anspruch 1, wonach die Formkörper aus verpreßtem Metall(hydr)oxidpulver eine innere Oberfläche von 30 bis 200 m²/g aufweisen.

5. Verfahren nach Anspruch 1, wonach der Wasserstoffdruck 100 bis 300 bar beträgt.

6. Verfahren nach Anspruch 1, wonach die Umlagerungstemperatur 220 bis 290°C beträgt.

7. Verfahren nach Anspruch 1, dadurch gekennzeichnet, daß während der Umlagerung des d-Menthols pro Mol Ausgangsmaterial mindestens die 5-fache molare Menge Wasserstoff den Reaktor passiert.

8. Verfahren nach Anspruch 1, dadurch gekennzeichnet, daß aus dem Reaktionsprodukt der Umlagerung von d-Menthol das d,l-Menthol destillativ entnommen wird und die restlichen Reaktionsprodukte in die Reaktion zurückgeführt werden.

## Claims

1. Continuous process for preparing d,l-menthol by catalytic rearrangement of optically active d-menthol in the presence of hydrogen under elevated pressure and at elevated temperature, characterized in that the rearrangement is carried out at a hydrogen pressure of from 50 to 350 bar and temperatures of from 200 to 350°C in a fixed-bed process over support-free shaped bodies which act as catalysts and are obtainable by reduction of shaped bodies of pressed powders of nickel, manganese and alkaline earth metal (hydr)oxides and optionally (hydr)oxides of elements of transition groups V and/or VI of the Periodic Table.

2. Process according to Claim 1, wherein the shaped bodies of pressed metal (hydr)oxide powders to be used for the reduction contain from 30 to 60% by weight of nickel, from 10 to 20% by weight of manganese, from 0.2 to 5% by weight of alkaline earth metal and up to 5% by weight of elements of transition groups V and/or VI of the Periodic Table (in each case calculated as metal), where the percentages are based on the total amount of metal (hydr)oxide powder mixture and the remainder to 100% by weight is oxygen.

3. Process according to Claim 1, wherein the shaped bodies of pressed metal (hydr)oxide powder have a compressive strength of from 300 to 800 N/cm² on the planar surface of the shaped body and from 50 to 200 N on the curved surface of the shaped body (measured in accordance with DIN 50 106).

4. Process according to Claim 1, wherein the shaped bodies of pressed metal (hydr)oxide powder have an internal surface area of from 30 to 200 m²/g.

5. Process according to Claim 1, wherein the hydrogen pressure is from 100 to 300 bar.

6. Process according to Claim 1, wherein the rearrangement temperature is from 220 to 290°C.

7. Process according to Claim 1, characterized in that during the rearrangement of the d-menthol at least a 5-fold molar amount of hydrogen per mol of starting material passes through the reactor.

8. Process according to Claim 1, characterized in that the d,l-menthol is taken by distillation from the reaction product of the rearrangement of d-menthol and the remaining reaction products are returned to the reaction.

## Revendications

1. Procédé continu pour la préparation du d,ℓ-menthol par transposition catalytique de l'isomère optique d-menthol en présence d'hydrogène sous pression et à haute température, caractérisé en ce que l'on procède à la transposition sous une pression d'hydrogène de 50 à 350 bar et à des températures de 200 à 350°C dans une opération en lit fixe sur des corps moulés sans support servant de catalyseurs qui ont été obtenus par réduction de corps moulés par compression de poudres d'(hydr)oxydes du nickel, du manganèse, de métaux alcalino-terreux et le cas échéant d'(hydr)oxydes d'éléments des sous-groupes V et/ou VI de la Classification Périodique.

2. Procédé selon la revendication 1, caractérisé en ce que les corps moulés par compression de poudres d'(hydr)oxydes métalliques utilisés pour la réduction contiennent de 30 à 60 % en poids de nickel, de 10 à 20 % en poids de manganèse, de 0,2 à 5 % en poids d'un métal alcalino-terreux et jusqu'à 5 % en poids d'éléments des sous-groupes V et/ou VI de la Classification Périodique (toutes ces indications exprimées en métal), ces pourcentages se rapportant à la quantité totale des poudres d'(hydr)oxydes métalliques et le complément à 100 % consistant en oxygène.

3. Procédé selon la revendication 1, dans lequel les corps moulés par compression de poudres d'(hydr)oxydes métalliques ont une résistance à la compression de 300 à 800 N/cm² sur la surface plane des corps moulés et de 50 à 200 N sur la surface courbe des corps moulés (mesures conformément à la norme allemande DIN 50 106).

4. Procédé selon la revendication 1, dans lequel les corps moulés par compression de poudres d'(hydr)oxydes métalliques ont une surface interne de 30 à 200 m²/g.

5. Procédé selon la revendication 1, dans lequel la pression d'hydrogène est de 100 à 300 bar.

6. Procédé selon la revendication 1, dans lequel la température de transposition est de 220 à 290°C.

7. Procédé selon la revendication 1, caractérisé en ce que, durant la transposition du d-menthol, on fait passer dans le réacteur de l'hydrogène en quantité au moins cinq fois molaire par rapport au composant de départ.

8. Procédé selon la revendication 1, caractérisé en ce que, à partir du produit de la réaction de transposition du d-menthol, on sépare le d,ℓ-menthol par distillation et on recycle le reste du produit de réaction dans les opérations.
